# EUROPEAN PATENT SPECIFICATION

(11) **EP 1 368 255 B1**
(45) Date of publication and mention of the grant of the patent: **29.11.2006**
(21) Application number: 02702569.1
(22) Date of filing: 11.03.2002
(51) Int. Cl.: B65D 83/38, A61M 15/00

(54) **CANISTERS FOR USE IN METERED DOSE INHALERS**
BEHAELTER FUER EINEN DOSIERINHALATOR
CARTOUCHE POUR AEROSOL-DOSEUR

(30) Priority: 12.03.2001 GB 0106046
(43) Date of publication of application: 10.12.2003
(73) Proprietor: GLAXO GROUP LIMITED, Greenford, Middlesex UB6 ONN (GB)
(72) Inventor: CHAN, Sandra, Liverpool, Merseyside L24 9JD (GB); BURT, Peter, Colin, Weston, Ware, Hertfordshire SG12 0DP (GB)
(74) Representative: Giddings, Peter John
(86) International application number: PCT/GB2002/001088
(87) International publication number: WO 2002/072449

(56) References cited:
- EP-A- 0 312 308
- WO-A-00/78286
- DE-A- 19 502 992
- FR-A- 2 205 886
- US-A- 6 019 252
- US-A- 6 131 566

## Description

This invention relates to an improved canister for use in metered dose inhalers (MDI's), especially to a canister which may contain and dispense pressurised formulations of pharmaceutical substances in propellants, for example, hydrofluoroalkane propellants.

Metered dose inhalers are a widely used system for delivery of pharmaceutical substances to the lung and upper airways. As such they have become established as a delivery system for pharmaceuticals for the treatment of respiratory disorders such as asthma and chronic obstructive pulmonary disease (COPD).

Metered dose inhalers typically comprise a canister fitted with a valve such as a metering valve which is fitted into a suitable channelling device to permit inhalation. Suitable channelling devices comprise, for example a valve actuator and a cylindrical or cone-like passage through which medicament may be delivered from the filled canister via the metering valve to the nose or mouth of a patient e.g. a mouthpiece actuator. In a typical arrangement the valve stem is seated in a nozzle block which has an orifice leading to an expansion chamber; the expansion chamber has an exit orifice which extends into the mouthpiece.

Metered dose inhalers operate by dispensing a metered volume of formulation by means of the metering valve from a bulk supply of formulation contained within the canister which generally consists of a suspension or solution of a substance in a liquefied propellant gas. In the past the preferred propellant gases were CFC's such as P11, P12 and 114. However, following the discovery that CFC's are capable of causing depletion of atmospheric zone alternative propellants have been developed. The currently most favoured alternative propellants are hydrofluoroalkanes, especially 1,1,1,2-tetrafluoroethane (HFA134a) and 1,1,1,2,3,3,3-heptafluoro-n-propane (HFA227).

The medical community has been engaged for many years now in the process of formulating old and new medicines in the alternative propellants. It is now generally recognised that because of certain chemical and physical differences between the old and new propellants certain other modifications to the MDI system have been considered advisable. For example, the surfactants previously commonly used as dispersing agents in suspension based CFC formulations are not effective since they are inadequately soluble in hydrofluoroalkanes without the addition of a co-solvent to the formulation. Efforts are being made to develop novel surfactants which are soluble in hydrofluoroalkanes (see e.g. WO96/09816 of Glaxo Inc). A system has also been developed in which the surfactant is omitted altogether (see e.g. WO93/11743 of Glaxo Group).

Certain suspension formulations of pharmaceutical products in hydrofluoroalkanes have demonstrated a tendency to suffer drug losses due to deposition of drug substances on the canister walls. Also the possibility of interaction between the formulation and the canister walls cannot always be excluded. For this reason canister coatings have been developed e.g. coatings of a fluorocarbon polymer optionally in combination with a non-fluorocarbon polymer (see e.g. WO96/32151 of Glaxo Wellcome Inc). Coatings of epoxy-phenol resins are described in WO00/30608 (Chiesi). WO00/78286 (3M) describes coatings including a thin film of glass. Processes employed in order to apply and cure such coatings which involve use of elevated temperature can cause distortion of conventional aluminium canisters. Accordingly aluminium canisters have been developed with certain strengthening features such as thicker walls and ellipsoidal base, however these are relatively expensive to manufacture.

US 6,131,566 (Glaxo Wellcome Inc.) discloses that strengthened MDI cans are capable of withstanding particularly stressful coating and curing conditions. Such strengthened cans may be made from strengthened aluminium or an aluminium alloy. They may also have side walls and a base of increased thickness or comprise a substantially ellipsoidal base.

Stainless steel is known as an alternative strong material for manufacture of canisters however lengths have to be taken to avoid corrosion e.g. as described in WO00/73170 (Boehringer Ingelheim) and use of special alloys is also expensive.

DE 195 02 992 (Brain Power Consulting GmbH) describes a container, for example an aerosol can, which is produced from strips of a flat material that surround the hollow space inside the container and are wound so as to overlap in an overlapping area which corresponds to at least one circumference of the substantially cylindrical outer surface.

We have now invented a canister suitable for use in metered dose inhalers which eliminates or substantially mitigates a number of the disadvantages of prior art canisters.

Thus according to the invention we provide a canister suitable for use in metered dose inhalers and fitted with a metering valve, characterised in that its walls are formed by deep drawing a laminate comprising steel sandwiched between two layers of aluminium and optionally the internal and/or the external aluminium walls are provided with one or more coating layers.

Such canisters are advantageous in that the laminate is capable of providing equivalent or greater strength than thicker walled aluminium cans at a more reasonable cost. Furthermore, problems associated with possible interaction between the metal and the contents of the can or the atmosphere may be avoided.
Laminate when used in this specification will be understood to mean a material comprising two or more layers each of which make a contribution to the structural integrity of the canister when attached together.

The laminate may optionally include one or more other layers such as:
i) a bonding layer e.g. a layer of adhesive such as an epoxy resin, thermoset adhesive or cyanoacrylate;
ii) a layer e.g. of a metal oxide which may be used to effect or improve adhesion between other layers such as between the metal layer and the strengthening layer;
iii) a coating layer e.g. applied to part or all of the internal and/or external surface of the laminate so as to serve one or more of the following purposes:
   a) reduction of interaction of a layer of laminate with the atmosphere (e.g. in the case of the coating layer applied to the external surface);
   b) reduction of interaction of a layer of laminate with the contents of the canister (e.g. in the case of the coating layer applied to the internal surface);
   c) reduction of deposition of particulate matter from the contents of the canister onto a layer of laminate (e.g. in the case of the coating layer applied to the internal surface).

The above optional layers may perform more than one function, for example, adhesives may also distribute stress at the bond point and/or resist moisture and or corrosion. Similarly the oxide layer may be resistant to corrosion. Alternatively, a coating layer applied to an internal surface may reduce the interaction between the layer of laminate and the contents of the canister and also reduce the deposition of particulate matter from the contents of the canister onto the internal surface of the laminate.

In a particularly preferred embodiment of the invention the wall of the canister is formed from a laminate comprising a layer of steel sandwiched between two layers of aluminium (i.e. is a laminate of one layer of steel and two layers of aluminium). Optionally the internal and/or external aluminium walls are provided with one or more coating layers. Preferably the internal wall of the canister will be coated with e.g. a fluorocarbon polymer coat such as PTFE or FEP; or a mixture of a fluorocarbon polymer with a non-fluorocarbon polymer such as PTFE or FEP and polyethersulphone; or an epoxy-phenol resin.

The steel for use according to the invention will generally consist of a ferrous alloy with greater than 50% iron and one or more of the following: 0.1 to 10 wt% carbon (C), 0 to 10 wt% manganese (Mn), 0 to 10 wt% copper (Cu), 0 to 10 wt% silicon (Si), 0 to 50 wt% nickel (Ni), 0 to 10 wt% vanadium (V), 0 to 10 wt% niobium (Nb), 0 to 10 wt% aluminium (Al), 0 to 10 wt% molybdenum (Mo), 0 to 50 wt% chromium (Cr), 0 to 5 wt% phosphorus (P), 0 to 5 wt% sulphur (S), 0 to 30 wt% tungsten (W), 0 to 20 wt% cobalt (Co), 0 to 10 wt% columbium (Cb), 0 to 10wt% oxygen (O), 0 to 10 wt% nitrogen (N) which may desirably be employed to improve the properties of the steel.

Stainless steel is a ferrous alloy as defined above which contains at least 12% chromium.

Where the steel is sandwiched between two layers of metals, preferably the steel used will be mild steel which is a ferrous alloy as defined above containing up to 2% carbon.

The total thickness of the laminate for the finished product is preferably in the range 0.2-2.5mm, especially 0.2-2.0mm. When the laminate comprises a first layer which is metal and a strengthening layer which is metal the thickness of each layer is independently in the range 0.1-1.0mm. When the laminate is a laminate of one layer of steel and one layer of aluminium or stainless steel the thickness of the layer is preferably in the range 0.1-1.0mm. When the laminate is a laminate of one layer of steel and two layers of aluminium (or one layer of aluminium and one layer of stainless steel) the thickness of the sheets is preferably in the range 0.1-1.0mm. When the laminate comprises a strengthening layer selected from a plastics material preferably the thickness of the said layer will be in the range 0.2-2.0mm especially, 0.8-1.5mm. The thickness of the laminate, or the constituent sheets thereof, may be reduced slightly with respect to the starting material when it is drawn into a canister.

As mentioned above, laminates may be prepared using an adhesive e.g. an epoxy resin with a molecular weight in the range 5000 to 30,000 (for further details see EP 0612 608). The adhesive will usually require curing e.g. by heating. Pressure sensitive adhesives (PSA) may also be used as an agent to bond the component layer together. The PSA may be activated by heating after assembly of the laminate and processing using pressure lamination rollers (for further details please see USP 3970 496). This method is especially suitable when the laminate is composed of at least two layers which are a metal e.g. wherein the lamina comprises a layer of aluminium or stainless steel and a layer of steel or a sandwich as described above.

Alternative methods of preparing laminates include: surface activation of some or all the component lamina e.g. by using materials which when subjected to a mechanical force, such as pressure rollers with raised portions, some molecules/atoms of the two materials migrate and bind the said materials together, alternatively the migration of some molecules/atoms of the two material may be promoted by heating; electro deposition e.g. electroplating with tin (for future details please see USP 5 298 149); or vapour deposition.

Canisters may be prepared from laminates by hot and/or cold drawing. For further information on these processes please see Materials Science and Engineering - an introduction by William D. Callister, Jr. (2nd edition), published by Wiley.

As mentioned above, canisters may be coated on their internal surface with a polymer e.g. a fluorocarbon polymer optionally in combination with a non-fluorocarbon polymer as described in WO 96/32151.

Suitable fluoropolymers include polytetrafluoroethylene (PTFE), ethylenetetrafluoroethylene (ETFE), polyvinyldienefluoride (PVDF), perfluoroalkoxyalkane (PFA), polyvinylfluoride (PVF), polychlorotrifluoroethylene (PCTFE) and fluorinated ethylenepropylene (FEP). Fluorocarbon polymers are marketed under trademarks such as Teflon^{®}, Tefzel^{®}, Halar^{®} and Hostaflon^{®}, Polyflon^{®} and Neoflon^{®}. Grades of polymer include FEP DuPont 856-200, PFA DuPont 857-200, PTFE-PES DuPont 3200-100, PTFE-FEP-polyamideimide DuPont 856P23485, FEP powder DuPont 532, and PFA Hoechst 6900n.

Fluorinated polymers which have a relatively high ratio of fluorine to carbon such as perfluorocarbon polymers e.g. PTFE, PFA and FEP are preferred.

Suitable copolymers comprise from 1 to 99%, preferably from 5 to 95% by weight of fluorinated polymer. Suitable copolymers include copolymers of tetrafluoroethylene (TFE) with PFA, TFE with hexafluoropropylene (HFP) (available as FEP 6107 and FEP 100 from DYNEON), VDF with HFP (commercially available as Viton A), TFE with perfluoro(propyl vinyl ether) (available as PFA 6515N from DYNEON), a blend of TFE, hexafluoropropylene and vinylidene fluoride (available commercially as THV 200G from DYNEON), HOSTAFORM X329^{™} (Hoechst) which is a 5% PTFE/Acetal blend, HOSTAFORM C9021TF which is a 20% PTFE/Acetal blend, PTFE/PBT blends (for example, LNP WL4040), and PTFE/PBT/silicone blends (for example, LNP WL4540).

The fluorinated polymer may be blended with non-fluorinated polymers such as polyamides, polyimides, polyamideimides, polyethersulphones, polyphenylene sulphides and amine-formaldehyde thermosetting resins. These added polymers improve adhesion to the canister walls, especially in the case of canisters lined with aluminium. Preferred polymer blends include PTFE/FEP/polyamideimide, PTFE/polyethersulphone and FEP/benzoguanamine.

The preferred polymer is a blend of polyethersulphone (PES) and polytetrafluoroethylene (PTFE). Another preferred polymer for coating is pure FEP (fluorinated ethylene propylene). Another preferred polymer for coating is pure PFA.

Canisters may be coated by the means known in the art for metal coating. For example, a metal such as aluminium or stainless steel may be pre-coated as a coil and cured before being stamped or drawn out into the can shape. Alternatively, preformed canisters may be sprayed inside with formulations of the coating polymer and then cured. The preformed canisters may also be dipped in the polymer coating formulation and cured, thus becoming coated on the inside and out. The polymer may also be formed in situ at the can walls using plasma polymerisation of the fluorocarbon monomers. The appropriate curing temperature is dependent on the polymer chosen for the coating and the coating method employed. However for coil coating and spray coating temperatures in excess of the melting point of the polymer are typically required, for example, about 50°C above the melting point, for up to 20 minutes. For the named preferred and particularly preferred polymers, curing temperatures in the range of around 300-400 °C e.g. around 350-380 °C are suitable.

Alternative coatings include: epoxy resin, phenolic resin, phenoxy resin, epoxy-phenol resin, epoxy-phenol-novolac obtained by glycidylation of phenolformaldehyde (novolac) condensation products, and epoxy-cresol-novolac obtained by glycidylation of o-cresol formaldehyde (o-cresol novolac) condensation products see WO00/30608.

Other suitable coatings comprise linear non-cross-linked fluorinated polymers. In one aspect, the coating compound comprises a functional grouping which is capable of anchoring the compound to the surface thereof. As a first example, the compound may be an organo-phosphate such as a phosphate based perfluoroether derivative.

Typically, the compound is a phosphoric ester.

In one first such embodiment, the interfacial surface has a compound disposed thereon having the general formula (I):

R¹ - (OC₃F₆)ₓ - (OCF₂)_{y} - R² (I)

wherein R¹ comprises a fluoro-alkyl functional group;
x and y are such that the molecular weight of the compound is in the range 350-1000; and
R² comprises a phosphoric ester functional group.

In a second such embodiment, the interfacial surface has a compound disposed thereon having the general formula (II):

R³-(CH₂)ᵥ-CF₂O-(C₂F₄O)ₓ - (CF₂O)_{y}CF₂-(CH₂)_{w}- R³ (II)

wherein R³comprises -(OCH₂-CH₂)₂-OPO(OH)₂;
x, y and z are such that the molecular weight of the compound is in the range 900-2100; and v and w independently represent 1 or 2.

In one preferred embodiment, v and w are both 1. In a second preferred embodiment v and w are both 2.

Alternatively in a second embodiment the compounds may be an organo-silane derivative such as a silane derivative of perfluoropolyoxyalkane, e.g. a silane derivative of perfluoropolyoxyalkane having a molecular weight in the range 1600-1750. Examples include perfluoropolyoxyalkanes having functional groups of the type -CONR⁴R⁵ wherein R⁴ and R⁵ may be independently selected from hydrogen, or a silyl ether (e.g. SiRₜ(OR)₃₋ₜ) wherein R=hydrogen or C₁₋₈alkyl and t=0 to 2) as described in USP 4 746 550 which is incorporated herein by reference.

The synthesis of compounds of formula (I) and (II) may readily be determined by reference to EP687533 which describes similar compounds. EP338531 also provides information on the preparation of compounds of this type. Methods of preparing organo-silane polymeric compounds of the type described above may readily be determined by reference to USP 4 746 550.

Whilst not wishing to be bound by any theory, it is believed that the phosphate or silane moiety of the compounds of formula as described above reacts with the surface of the component to anchor the compound to the surface. Thus, when in use, the per-fluorinated end of the compound is presented to the pharmaceutical formulation and so provides a highly fluorinated surface.

Other suitable coatings include siloxanes such as dimethyl siloxane which in one aspect, may be applied by plasma polymerisation processes.

One suitable means of applying a fluorine-containing coating is by plasma coating, for example, by a CF₄ or fluorine ion plasma coating technique. The plasma coating may consist of a fluorinated polymer laid down on the surface of the component by polymerisation or by modification of a hydrocarbon-containing pre-coating on the surface by interchange of hydrogen ions in the material with fluorine ions. The coating process typically takes place in a vacuum at ambient temperature. The components to be coated are placed inside a chamber which is evacuated. The fluorine monomer or fluorine source is introduced into the chamber at a controlled rate. The plasma is ignited within the chamber and maintained for a given time at a chosen power setting. For plasma polymerization typically temperatures in the range of about 20°C to about 100°C may be employed. At the end of the treatment the plasma is extinguished, the chamber flushed and the products retrieved. In the polymerisation process, a thin layer of plasma polymer will be bonded to the surface.

In one embodiment the internal wall of the canister may comprise a coating layer of high-nitryl resin which is a co-polymer comprising an unsaturated nitryl compound e.g. acrylonitrile or methacrylonitrile and an unsaturated copolymer e.g. an unsaturated aromatic compound, a diene compound an unsaturated ether compound or an unsaturated ester such as styrene, α-methylstyrene, butadiene, isoprene, methylacrylate, ethylacrylate, methylmethacrylate and ethylmethacrylate, wherein said co-polymer contains greater than 50 percent by weight of the unsaturated nitryl unit. The nitryl resin layer may be manufactured by methods such as multilayer blow moulding, multilayer injection-blow moulding and multilayer injection moulding. Optionally an adhesive layer may be used between the nitryl resin layer and the metal layer. The nitryl resin layer has the advantage of being resistant to chemical attack and strong.

An alternative coating is a thin layer of glass which may be deposited by gas vapour deposition on the internal wall of the canister.

The coating thickness will typically be in the range 0.1 micron to 1 mm, e.g. 1-100 microns especially 1 to 25 microns.

Coatings may be applied in one or more coats.

Use of coatings, for example, plastics coatings may not be necessary if the surface, especially the internal surfaces of the canister are non-metallic, for example, a plastics material.

In use, the canister will be fitted with a valve, e.g. a metering valve, usually located in a cap covering the mouth of the canister. The metering valve is designed to deliver a metered amount of the formulation per actuation and incorporate a gasket to prevent leakage of propellant through the valve. Suitable valves are commercially available from manufacturers well known in the aerosol industry, for example, from Valois, France (eg. DF10, DF30, DF60), Bespak plc, UK (e.g. BK300, BK356, BK357) and 3M-Neotechnic Ltd, UK (eg. Spraymiser^{™}). The DF31 valve of Valois, France is also suitable.

Valve metering volumes of 25 µl, 50 µl, 63 µl or 100 µl are typical.

Other valve systems include, but are not limited to, wedge gate valve systems, double-disc gate valve systems, globe and angle valve systems, swing check valve systems, end cock valve systems, and other like valve systems.

Valve materials, especially the metering chamber, will preferably be manufactured of a material which is inert to and resists distortion by the contents of the formulation, especially when the contents include ethanol. Particularly suitable materials for use in manufacture of the metering chamber include polyesters e.g. polybutyleneterephthalate (PBT) and acetals, especially PBT.

Materials for use in the manufacture of the metering chamber and/or the valve stem may desirably be fluorinated, partially fluorinated or impregnated with fluorine containing substances in order to resist drug deposition.

Valve seals, especially the gasket seal, and also the seals around the metering chamber, will preferably be manufactured of a material which is inert to and resists extraction into the contents of the formulation, especially when the contents include ethanol.

Gaskets may comprise any suitable elastomeric material such as, for example, low density polyethylene, chlorobutyl, black and white butadiene-acrylonitrile rubbers, butyl rubber, neoprene, EPDM (a polymer of ethylenepropylenediene monomer) (eg as described in WO95/02651) and TPE (thermoplastic elastomer; eg as described in WO92/11190).

The elastomeric material may either comprise a thermoplastic elastomer (TPE) or a thermoset elastomer which may optionally be cross-linked.

Typically, the sealing ring, which seals the valve to the can, and/or second sealing ring comprise an elastomeric material. The ring is typically resiliently deformable.

The sealing ring may be formed by cutting a ring from a sheet of suitable material. Alternatively, the sealing ring may be formed by a moulding process such as an injection moulding, a compression moulding or a transfer moulding process.

The sealing ring may also comprise a thermoplastic elastomer blend or alloy in which an elastomeric material is dispersed in a thermoplastic matrix. The elastomers may optionally additionally contain conventional polymer additives such as processing aids, colorants, tackifiers, lubricants, silica, talc, or processing oils such as mineral oil in suitable amounts.

Suitable thermoset rubbers include butyl rubbers, chloro-butyt rubbers, bromo-butyl rubbers, nitrile rubbers, silicone rubbers, fluorosilicone rubbers, fluorocarbon rubbers, polysulphide rubbers, polypropylene oxide rubbers, isoprene rubbers, isoprene-isobutene rubbers, isobutylene rubbers or neoprene (polychloroprene) rubbers.

Suitable thermoplastic elastomers comprise a copolymer of about 80 to about 95 mole percent ethylene and a total of about 5 to about 20 mole percent of one or more co-monomers selected from the group consisting of 1-butene, 1-hexene, and 1-octene as known in the art. Two or more such copolymers may be blended together to form a thermoplastic polymer blend.

Another suitable class of thermoplastic elastomers are the styrene-ethylene/butylene-styrene block copolymers. These copolymers may additionally comprise a polyolefin (e.g. polypropylene) and a siloxane.

Thermoplastic elastomeric material may also be selected from one or more of the following: polyester rubbers, polyurethane rubbers, ethylene vinyl acetate rubber, styrene butadiene rubber, copolyether ester TPE, olefinic TPE, polyester amide TPE and polyether amide TPE.

Other suitable elastomers include ethylene propylene diene rubber (EPDM). The EPDM may be present on its own or present as part of a thermoplastic elastomer blend or alloy, e.g. in the form of particles substantially uniformly dispersed in a continuous thermoplastic matrix (e.g. polypropylene or polyethylene). Commercially available thermoplastic elastomer blend and alloys include the SANTOPRENE^{™} elastomers. Other suitable thermoplastic elastomer blends include butyl-polyethylene (e.g. in a ratio ranging between about 2:3 and about 3:2) and butyl-polypropylene.

Typically, the sealing ring and/or the second sealing ring additionally comprises lubricant material. Suitably, the sealing ring and/or the second sealing ring comprises up to 30%, preferably from 5 to 20% lubricant material.

In addition, the stem may also comprise lubricant material. Suitably, the valve stem comprises up to 30%, preferably from 5 to 20% lubricant material.

The term 'lubricant' herein means any material which reduces friction between the valve stem and seal. Suitable lubricants include silicone oil or a fluorocarbon polymer such as polytetrafluoroethane (PTFE) or fluoroethylene propylene (FEP).

Lubricant can be applied to the stem, sealing ring or a second sealing ring by any suitable process including coating and impregnation, such as by injection or a tamponage process.

Optionally a moisture absorbing means may be incorporated within the dispenser as a component thereof. Alternatively, the moisture absorbing means may be a separate component of the formulation contained within the dispenser,

The moisture absorbing means may comprise a component or accessory for use with a canister or valve including a desiccant such as a molecular sieve, silica gel and/or a component or accessory that is made from a plastics material which is a natural desiccant, such as a polyamide, for example nylon, or may be moulded from other plastics material such as acetal or PBT. Alternatively, or in addition, the moisture absorbing means may comprise an internal lining or coating. In one embodiment, the moisture absorbing means may be incorporated into a treatment or coating for canisters and/or valves for preventing drug deposition and/or maintaining dose uniformity.

Other vapour or moisture absorbing materials include desiccants made from inorganic materials such zeolites and aluminas. Such inorganic materials have high water absorption capacities and favourable water absorption isotherm shapes. The water absorption capacity of such materials typically varies from 20 to 50 weight percent.

Other exemplary moisture absorbing materials include, but are not limited to, alumina, bauxite, anhydrous calcium sulphate, water-absorbing Clay, activated bentonite clay, molecular sieves, or other like materials.

In conjunction with the desiccant an additional compound may be added to act as a conduit/channelling agent to increase/optimise the efficiency of the moisture absorption properties. Such materials may include compounds such as polyethylene glycols.

Preferably, the means for absorbing moisture reduces the rise in moisture content over time, and/or the decrease in Fine Particulate Mass (FPM) over time by between 20 and 100%, for example, 40 to 70%, e.g. 45 to 55%. Typically, the component or accessory takes the form of a cap and/or a seal and/or a lining.

Furthermore desiccant material may be included in the packaging material for the device.

The desiccant should be present in an amount sufficient to absorb any increases in moisture around the valve area of the MDI and thus alleviate or substantially prevent moisture increases inside the canister.

Typically, 100µg to 5g, for example, 1 mg to 1g, e.g. 100mg to 500mg, such as about 100mg to 250mg of desiccant may be included.

Canisters according to the invention may be filled with pharmaceutical formulations suitable for administration to patients e.g. those suffering from respiratory disorders such as asthma and COPD. Suitable formulations generally comprise a liquefied propellant gas such as a hydrofluoroalkane e.g. HFA134a or HFA227 and one or more drug substances.

Thus provided is a canister according to the invention containing a formulation comprising a medicament and a hydrofluoroalkane propellant Preferably the propellant is selected from 1,1,1,2-tetrafluoroethane, 1,1,1,2,3,3,3-hepafluoro-n-propane and mixtures thereof, especially 1,1,1,2-tetrafluoroethane.

Preferably the drug will be in particulate form of a mass median diameter so as to permit inhalation into the bronchial airways i.e. generally less than 100 microns e.g. between around 1 and 10 microns especially 1-5 microns. Particle size reduction can be achieved e.g. by micronisation. Optionally the formulation may contain a dispersing agent and/or a co-solvent such as a C₂₋₆ aliphatic alcohol (e.g. ethanol) or a polyol (e.g. propylene glycol or polyethyleneglycol), especially ethanol. A suitable amount of dispersing agent would be around 0.001-50%, e.g. 0.05-5% by weight of drug. A suitable amount of co-solvent would be around 0.005-15% e.g. 0.1-5% by weight of formulation. When conventional dispersing agents such as lecithin, sorbitan trioleate and oleic acid are employed then ethanol will generally be employed as well. Alternatively, the drug may be dissolved in the liquefied propellant gas, generally by use of a co-solvent such as a C₂₋₆ aliphatic alcohol (e.g. ethanol) or a polyol (e.g. propylene glycol or polyethyleneglycol), especially ethanol. Certain preferred suspension formulations are free of all excipients besides the propellant and the particulate drug. Such preferred formulations consist only of particulate drug and propellant.

Example medicaments may thus be selected from, for example, analgesics, e.g. codeine, dihydromorphine, ergotamine, fentanyl or morphine; anginal preparations, e.g. diltiazem; antiallergics, e.g. cromoglycate (e.g. s the sodium salt), ketotifen or nedocromil (e.g. as the sodium salt); anti-infectives e.g. cephalosporins, penicillins, streptomycin, sulphonamides, tetracyclines and pentamidine; antihistamines, e.g. methapyrilene; anti- inflammatories, e.g. beclomethasone (e.g. as the dipropionate ester), fluticasone (e.g. as the propionate ester), flunisolide, budesonide, rofleponide, mometasone e.g. as the furoate ester), ciclesonide, triamcinolone (e.g. as the acetonide) or 6α, 9α-difluoro-11β-hydroxy-16α-methyt-3-oxo-17α-propionyloxy-androsta-1,4-diene-17β-carbothioic acid S-(2-oxo-tetrahydro-furan-3-yl) ester; antitussives, e.g. noscapine; bronchodilators, e.g. albuterol (e.g. as free base or sulphate), salmeterol (e.g. as xinafoate), ephedrine, adrenaline, fenoterol (e.g. as hydrobromide), formoterol (e.g. as fumarate), isoprenaline, metaproterenol, phenylephrine, phenylpropanolamine, pirbuterol (e.g. as acetate), reproterol (e.g. as hydrochloride), rimiterol, terbutaline (e.g. as sulphate), isoetharine, tulobuterol or 4-hydroxy-7-[2-[[2-[[3-(2-phenylethoxy)propyl]sulfonyl]ethyl]amino]ethyl-2(3H)-benzothiazolone, diuretics, e.g. amiloride; anticholinergics, e.g. ipratropium (e.g. as bromide), tiotropium, atropine or oxitropium: hormones, e.g. cortisone, hydrocortisone or prednisolone; xanthines, e.g. aminophylline, choline theophyllinate, lysine theophyllinate or theophylline. It will be dear to a person skilled in the art that, where appropriate, the medicaments may be used In the form of salts, (e.g. as alkali metal or amine salts or as acid addition salts) or as esters (e.g. lower alkyl esters) or as solvates (e.g. hydrates) to optimise the activity and/or stability of the medicament and/or to minimise the solubility of the medicament in the propellant.

Preferred medicaments are selected from albuterol, salmeterol, fluticasone propionate and beclomethasone dipropionate and salts or solvates thereof, e.g. the sulphate of albuterol and the xinafoate of salmeterol and mixtures thereof.

Medicaments can also be delivered in combinations. Preferred formulations containing combinations of active ingredients contain salbutamol (e.g. as the free base or the sulphate salt) or salmeterol (e.g. as the xinafoate salt) or formoterol (e.g. as the fumarate salt) in combination with an anti-inflammatory steroid such as a beclomethasone ester (e.g. the dipropionate) or a fluticasone ester (e.g. the propionate) or budesonide. A particularly preferred combination is a combination of fluticasone propionate and salmeterol, or a salt thereof (particularly the xinafoate salt). A further combination of particular interest is budesonide and formoterol (e.g. as the fumarate salt).

The final drug concentration in the formulation is preferably between 0.005-10% w/w more preferably 0.005-5% e.g. 0.01-1% w/w by weight of formulation. Preferably the concentration will be such as to deliver a therapeutic dose of the medicament in one or two actuations. Example therapeutic doses are 25, 50, 125 or 250µg per actuation of fluticasone propionate taken twice per day, 25µg per actuation of salmeterol (as xinafoate) taken twice per day, 100µg of salbutamol (e.g. as free base or sulphate) taken twice as needed and 50 or 250 µg per actuation of beclomethasone dipropionate taken twice two or three times per day.

The invention also provides use of canisters according to the invention in inhalation therapy, for example, for the treatment or prophylaxis of respiratory disorders; and use of a metered dose inhaler system in the treatment or prophylaxis of respiratory disorders are all alternative aspects of this invention.

A still further aspect of the present invention comprises a method of treating respiratory disorders such as, for example, asthma, which comprises administration by inhalation of an effective amount of a pharmaceutical formulation from a canister according to the invention.

The invention is illustrated by reference to the following examples.

### Example 1

A laminate comprising two sheets of aluminium and a sheet of stainless steel each of thickness 0.2mm is formed using a pressure sensitive adhesive, heating and pressure lamination rollers, so as to form a sandwich of a steel layer between two aluminium layers. The laminate is deep drawn into the shape of a conventional MDI canister (approximate dimensions: diameter 22.1-22.2 mm, height 33.4-61.15mm; wall thickness 0.3-0.75 mm).

### Example 2

The canister of Example 1 is spray coated on its internal surface with a blend of PTFE and PES and cured at between 350 to 400°C for approximately 10 minutes.

### Example 3

The canister of Example 1 is spray coated on its internal surface with FEP and cured 350 to 400°C for approximately 10 minutes.

### Example 4

The canisters of Examples 1, 2 and 3 may be fitted with a metering valve (Valois DF60) and filled through the valve with a suspension of fluticasone propionate in liquefied HFA134a.

### Example 5

The canisters of Examples 1, 2 and 3 may be fitted with a metering valve (Valois DF60) and filled through the valve with a suspension of salmeterol xinafoate in liquefied HFA134a.

### Example 6

The canisters of Examples 1, 2 and 3 may be fitted with a metering valve (Valois DF60) and filled through the valve with a suspension of salbutamol sulphate in liquefied HFA134a.

### Example 7

The canisters of Examples 1, 2 and 3 may be fitted with a metering valve (Valois DF60) and filled through the valve with a suspension of fluticasone propionate and salmeterol xinafoate in liquefied HFA134a.

Throughout the specification and the claims which follow, unless the context requires otherwise, the word 'comprise', and variations such as 'comprises' and 'comprising', will be understood to imply the inclusion of a stated integer or step or group of integers but not to the exclusion of any other integer or step or group of integers or steps.

## Claims

1. A canister suitable for use in metered dose inhalers and fitted with a metering valve, **characterised in that** its walls are formed by deep drawing a laminate comprising steel sandwiched between two layers of aluminium and optionally the internal and/or external aluminium walls are provided with one or more coating layers.

2. A canister according to claim 1 wherein the total thickness of the laminate is in the range 0.2 to 2.5 mm.

3. A canister according to claims 1 or 2 wherein the total thickness of the laminate is in the range 0.2 to 2.0 mm.

4. A canister according to claim 1 wherein the thickness of each layer is in the range 0.1 to 1.0 mm.

5. A canister according to of any claims 1-4 wherein the canister further comprises a first layer of adhesive between the first aluminium layer and the steel layer and a second layer of adhesive between the second aluminium layer and the steel layer.

6. A canister according to claim 5 wherein the first layer of adhesive is a pressure sensitive adhesive.

7. A canister according to daim 5 wherein the first and second layers of adhesive are pressure sensitive adhesive.

8. A canister according to any one of claims 1 to 7 containing a formulation comprising medicament and hydrofluoroalkane propellant.

9. A canister according to claim 8 wherein the propellant is selected from 1,1,1,2-tetrafluoroethane, 1,1,1,2,3,3.3-heptafluoro-n-propane and mixtures thereof.

10. A canister according to claim 9 wherein the propellant is 1,1,1,2-tetrafluoroethane.

11. A canister according to any one of claims 8 to 10 wherein the formulation is a suspension formulation free of all excipients besides propellant and the particulate drug.

12. A canister according to any one of claims 8 to 11 wherein the medicament is selected from fluticasone propionate, beclomethasone dipropionate, salmeterol, albuterol and salts or solvates thereof and mixtures thereof.

13. A canister according to claim 12 wherein the medicament is fluticasone propionate in combination with salmeterol xinafoate.

## Patentansprüche

1. Behälter, geeignet zur Verwendung in Dosierinhalatoren und ausgestattet mit einem Dosierventil, **dadurch gekennzeichnet, daß** seine Wandungen durch Tiefziehen eines Laminates gebildet werden, das Stahl umfaßt, der zwischen zwei Schichten aus Aluminium gelegen ist, und gegebenenfalls die internen und/oder externen Aluminiumwandungen mit einer oder mehreren Überzugsschichten versehen sind.

2. Behälter nach Anspruch 1, worin die Gesamtdicke des Laminats im Bereich 0,2 bis 2,5 mm ist.

3. Behälter nach Ansprüchen 1 oder 2, worin die Gesamtdicke des Laminats im Bereich 0,2 bis 2,0 mm ist.

4. Behälter nach Anspruch 1, worin die Dicke jeder Schicht im Bereich 0,1 bis 1,0 mm ist.

5. Behälter nach einem der Ansprüche 1 bis 4, worin der Behälter weiter eine erste Klebstoffschicht zwischen der ersten Aluminiumschicht und der Stahlschicht und eine zweite Klebstoffschicht zwischen der zweiten Aluminiumschicht und der Stahlschicht umfaßt.

6. Behälter nach Anspruch 5, worin die erste Klebstoffschicht ein Haftkleber ist.

7. Behälter nach Anspruch 5, worin die ersten und die zweiten Klebstoffschichten ein Haftkleber sind.

8. Behälter nach einem der Ansprüche 1 bis 7, der eine Formulierung enthält, die Medikament und Fluorwasserstoffalkan-Treibmittel umfaßt.

9. Behälter nach Anspruch 8, worin das Treibmittel ausgewählt ist aus 1,1,1,2-Tetrafluorethan, 1,1,1,2,3,3,3-Heptafluor-n-propan und Mischungen davon.

10. Behälter nach Anspruch 9, worin das Treibmittel 1,1,1,2-Tetrafluorethan ist.

11. Behälter nach einem der Ansprüche 8 bis 10, worin die Formulierung eine Suspensionsforumulierung ist, die abgesehen von Treibmittel und dem teilchenförmigen Arzneimittel, frei von allen Hilfsstoffen ist.

12. Behälter nach einem der Ansprüche 8 bis 11, worin das Medikament ausgewählt ist aus Fluticasonpropionat, Beclomethasondipropionat, Salmeterol, Albuterol und Salzen oder Solvaten und Mischungen davon.

13. Behälter nach Anspruch 12, worin das Medikament Fluticasonpropionat in Kombination mit Salmeterolxinafoat ist.

## Revendications

1. Cartouche adaptée à une utilisation dans des aérosols-doseurs et dotée d'une valve de dosage, **caractérisée en ce que** ses parois sont formées par emboutissage profond d'un laminé comprenant de l'acier en sandwich entre deux couches d'alum inium et éventuellement, les parois d'aluminium interne et/ou externe sont recouvertes d'une ou plusieurs couches de revêtement.

2. Cartouche selon la revendication 1, dans laquelle l'épaisseur totale du laminé est dans la plage de 0,2 à 2,5 mm.

3. Cartouche selon les revendications 1 ou 2, dans laquelle l'épaisseur totale du laminé est dans la plage 0,2 à 2,0 mm.

4. Cartouche selon la revendication 1, dans laquelle l'épaisseur de chaque couche est dans la plage de 0,1 à 1,0 mm.

5. Cartouche selon l'une que lconque des revendications 1 à 4, dans laquelle la cartouche comprend en outre une première couche d'adhésif entre la première couche d'aluminium et la couche d'acier et une seconde couche d'adhésif entre la seconde couche d'aluminium et la couche d'acier.

6. Cartouche selon la revendication 5, dans laquelle la première couche d'adhésif est un adhésif sensible à la pression.

7. Cartouche selon la revendication 5, dans laquelle les première et seconde couches d'adhésif sont un adhésif sensible à la pression.

8. Cartouche selon l'une quelconque des revendications 1 à 7, contenant une formule comprenant un médicament et un agent propulseur hydrofluoroalcane.

9. Cartouche selon la revendication 8, dans laquelle l'agent propulseur est choisi parmi le 1,1,1,2-tétrafluoroéthane, le 1,1,1,2,3,3,3-heptafluoro-n-propane et des mélanges de ceux-ci.

10. Cartouche selon la revendication 9, dans laquelle l'agent propulseur est du 1,1,1,2-tétrafluoroéthane.

11. Cartouche selon l'une quelconque des revendications 8 à 10, dans laquelle la formule est une formule de suspension exempte de tout excipient autre que l'agent propulseur et le médicament en poudre.

12. Cartouche selon l'une quelconque des revendications 8 à 11, dans laquelle le médicament est choisi parmi le propionat e de fluticasone, le dipropionate de beclométhasone, le salmétérol, l'albutérol et des sels ou solvates de ceux-ci et mélanges de ceux-ci.

13. Cartouche selon la revendication 12, dans laquelle le médicament est un propionate de fluticasone combiné à un xinafoate de salmétérol.
